# EUROPÄISCHE PATENTSCHRIFT

(11) **EP 1 481 004 B1**
(45) Veröffentlichungstag und Bekanntmachung des Hinweises auf die Patenterteilung: **02.12.2009**
(21) Anmeldenummer: 03718606.1
(22) Anmeldetag: 20.02.2003
(51) Int. Cl.: C07K 7/00, C07K 14/00, C07K 19/00, G01N 33/53, C12N 15/10

(54) **STREPTAVIDIN-BINDUNGSPEPTID**
STREPTAVIDIN-BINDING PEPTIDE
PEPTIDE DE LIAISON A LA STREPTAVIDINE

(30) Priorität: 01.03.2002 DE 10208877; 16.10.2002 DE 10248318
(43) Veröffentlichungstag der Anmeldung: 01.12.2004
(60) Teilanmeldung: 05090181.8
(73) Patentinhaber: Erdmann, Volker A., 14163 Berlin (DE); Lamla, Thorsten, 14195 Berlin (DE)
(72) Erfinder: Erdmann, Volker A., 14163 Berlin (DE); Lamla, Thorsten, 14195 Berlin (DE)
(74) Vertreter: Jungblut, Bernhard Jakob
(86) Internationale Anmeldenummer: PCT/DE2003/000605
(87) Internationale Veröffentlichungsnummer: WO 2003/074546

(56) Entgegenhaltungen:
- WO-A-01/01748
- WO-A-01/90331
- WO-A-99/57565
- US-A- 5 506 121
- US-B1- 6 342 581
- YAMAKI E A: "High-performance liquid chromatography of peptides on a mocrospherical carbon column" JOURNAL OF CHROMATOGRAPHY A, ELSEVIER SCIENCE, NL, Bd. 729, 1996, Seiten 143-153, XP002095513 ISSN: 0021-9673
- VELAZQUEZ C ET AL: "Quantitation of lysozyme peptides bound to class II MHC molecules indicates very large differences in levels of presentation." JOURNAL OF IMMUNOLOGY (BALTIMORE, MD.: 1950) UNITED STATES 1 MAY 2001, Bd. 166, Nr. 9, 1. Mai 2001 (2001-05-01), Seiten 5488-5494, XP002259794 ISSN: 0022-1767
- KAY B K ET AL: "An M13 phage library displaying random 38-amino-acid peptides as a source of novel sequences with affinity to selected targets" GENE, ELSEVIER BIOMEDICAL PRESS. AMSTERDAM, NL, Nr. 128, 1993, Seiten 59-65, XP002074893 ISSN: 0378-1119
- LAMLA T ET AL: "In vitro selection of other proteins than antibodies by means of ribosome display" FEBS LETTERS, ELSEVIER SCIENCE PUBLISHERS, AMSTERDAM, NL, Bd. 502, Nr. 1-2, 27. Juli 2001 (2001-07-27), Seiten 35-40, XP004296815 ISSN: 0014-5793
- ZANG X ET AL: "Tight-binding streptavidin ligands from a cyclic peptide library" BIOORGANIC & MEDICINAL CHEMISTRY LETTERS, OXFORD, GB, Bd. 8, Nr. 17, 8. September 1998 (1998-09-08), Seiten 2327-2332, XP004138226 ISSN: 0960-894X
- OESTERGAARD S ET AL: "NOVEL AVIDIN AND STREPTAVIDIN BINDING SEQUENCES FOUND IN SYNTHETIC PEPTIDE LIBRARIES" FEBS LETTERS, Bd. 362, 1995, Seiten 306-308, XP002949149 ISSN: 0014-5793
- DEVLIN J J ET AL: "RANDOM PEPTIDE LIBRARIES: A SOURCE OF SPECIFIC PROTEIN BINDING MOLECULES" SCIENCE, AMERICAN ASSOCIATION FOR THE ADVANCEMENT OF SCIENCE,, US, Bd. 249, 27. Juli 1990 (1990-07-27), Seiten 404-406, XP000616029 ISSN: 0036-8075
- WILSON DAVID S ET AL: "The use of mRNA display to select high-affinity protein-binding peptides" PROCEEDINGS OF THE NATIONAL ACADEMY OF SCIENCES OF USA, NATIONAL ACADEMY OF SCIENCE. WASHINGTON, US, Bd. 98, Nr. 7, 27. März 2001 (2001-03-27), Seiten 3750-3755, XP002209215 ISSN: 0027-8424
- SCHMIDT THOMAS G M ET AL: "Molecular interaction between the Strep-tag affinity peptide and its cognate target, streptavidin" JOURNAL OF MOLECULAR BIOLOGY, LONDON, GB, Bd. 255, Nr. 5, 1996, Seiten 753-766, XP002209213 ISSN: 0022-2836
- LAMLA T ET AL: "Searching Sequence Space for High-affinity Binding Peptides using Ribosome Display" JOURNAL OF MOLECULAR BIOLOGY, LONDON, GB, Bd. 329, Nr. 2, 30. Mai 2003 (2003-05-30), Seiten 381-388, XP004454265 ISSN: 0022-2836

### SEQUENCE LISTING

<110> Erdmann, Volker A.
   Lamla, Thorsten
<120> Steptavidin-Bindungspeptid
<130> ERD/PCT/0303
<150> DE 10208877
   <151> 2002-03-01
<150> DE 10248318
   <151> 2002-10-16
<160> 17
<170> PatentIn version 3.1
<210> 1
   <211> 5
   <212> PRT
   <213> artificial
<220>
   <221> misc_feature
   <222> (1)..(5)
   <223>
<400> 1
   Asp Val Glu Ala Trp
   1               5
<210> 2
   <211> 4
   <212> PRT
   <213> artificial
<220>
   <221> misc_feature
   <222> (1)..(4)
   <223>
<400> 2
   Asp Val Glu Ala
   1
<210> 3
   <211> 4
   <212> PRT
   <213> artificial
<220>
   <221> misc_feature
   <222> (1)..(4)
   <223>
<400> 3
   Val Glu Ala Trp
   1
<210> 4
   <211> 3
   <212> PRT
   <213> artificial
<220>
   <221> misc_feature
   <222> (1)..(3)
   <223>
<400> 4
   Asp Val Glu
   1
<210> 5
   <211> 3
   <212> PRT
   <213> artificial
<220>
   <221> misc_feature
   <222> (1)..(3)
   <223>
<400> 5
   Val Glu Ala
   1
<210> 6
   <211> 3
   <212> PRT
   <213> artificial
<220>
   <221> misc_feature
   <222> (1)..(3)
   <223>
<400> 6
   Glu Ala Trp
   1

<210> 7
   <211> 5
   <212> PRT
   <213> artificial
<220>
   <221> misc_feature
   <222> (3)..(3)
   <223> beliebige Aminosäure, insbesondere E, D, oder G
<400> 7
   Asp Val Xaa Ala Trp
   1 5
<210> 8
   <211> 6
   <212> PRT
   <213> artificial
<220>
   <221> misc_feature
   <222> (3)..(3)
   <223> beliebige Aminosäure, insbesondere E, D oder G
<400> 8
   Asp Val Xaa Ala Trp Leu
   1 5
<210> 9
   <211> 7
   <212> PRT
   <213> artificial
<220>
   <221> misc_feature
   <222> (3)..(3)
   <223> beliebige Aminosäure, insbesondere E, D oder G
<220>
   <221> misc_feature
   <222> (7)..(7)
   <223> beliebige Aminosäure, insbesondere D oder G
<400> 9
   Asp Val Xaa Ala Trp Leu Xaa
   1 5
<210> 10
   <211> 8
   <212> PRT
   <213> artificial
<220>
   <221> misc_feature
   <222> (3)..(3)
   <223> beliebige Aminosäure, insbesondere E, D oder G
<220>
   <221> misc_feature
   <222> (7)..(7)
   <223> beliebige Aminosäure, insbesondere D oder G
<220>
   <221> misc_feature
   <222> (8)..(8)
   <223> beliebige Aminosäure, insbesondere A oder E
<400> 10
   Asp Val Xaa Ala Trp Leu Xaa Xaa
   1 5
<210> 11
   <211> 9
   <212> PRT
   <213> artificial
<220>
   <221> misc_feature
   <222> (3)..(3)
   <223> beliebige Aminosäure, insbesondere E, D, oder G
<220>
   <221> misc_feature
   <222> (7)..(7)
   <223> beliebige Aminosäure, insbesondere D oder G
<220>
   <221> misc_feature
   <222> (8)..(8)
   <223> beliebige Aminosäure, insbesondere A oder E
<400> 11
   Asp Val Xaa Ala Trp Leu Xaa Xaa Arg
   1 5
<210> 12
   <211> 15
   <212> PRT
   <213> artificial
<220>
   <221> misc_feature
   <222> (3)..(3)
   <223> beliebige Aminosäure, insbesondere E, D oder G
<220>
   <221> misc_feature
   <222> (7)..(7)
   <223> beliebige Aminosäure, insbesondere D oder G
<220>
   <221> misc_feature
   <222> (8)..(8)
   <223> beliebige Aminosäure, insbesondere A oder E
<400> 12
   Asp Val Xaa Ala Trp Leu Xaa Xaa Arg Val Pro Leu Val Glu Thr
   1 5 10 15
<210> 13
   <211> 6
   <212> PRT
   <213> artificial
<220>
   <221> misc_feature
   <222> (1)..(6)
   <223> carboxyterminal an Seq.-ID 11 anschliessende Sequenz mit 1 - 6 de r hier definierten Aminosäuren, beginnend aminoterminal
<400> 13
   Val Pro Leu Val Glu Thr
   1 5
<210> 14
   <211> 15
   <212> PRT
   <213> artificial
<220>
   <221> misc_feature
   <222> (1)..(15)
   <223>
<400> 14
   Asp Leu Tyr Asp Ile Asp Arg Asn Trp Val Gly His Pro Gln Gly
   1 5 10 15
<210> 15
   <211> 15
   <212> PRT
   <213> artificial
<220>
   <221> misc_feature
   <222> (1)..(15)
   <223>
<400> 15
   Asp Asn Tyr Asp Ala Asp Leu Ala Trp Asp Thr His Pro Gln Asp
   1 5 10 15
<210> 16
   <211> 15
   <212> PRT
   <213> artificial
<220>
   <221> misc_feature
   <222> (1)..(15)
   <223>
<400> 16
   Asp Val Glu Ala Trp Leu Asp Glu Arg Val Pro Leu Val Glu Thr
   1 5 10 15
<210> 17
   <211> 15
   <212> PRT
   <213> artificial
<220>
   <221> misc_feature
   <222> (1)..(15)
   <223>
<400> 17
   Asp Val Glu Ala Trp Ile Ala Asp Pro Ala Val His Phe Thr Thr
   1               5                   10                  15

## Beschreibung

### Gebiet der Erfindung

Die Erfindung betrifft ein Streptavidin-Bindungspeptid, sowie Verfahren zur Herstellung eines Streptavidin-Bindungspeptides in einem zellbasierten oder zellfreien Proteinbiosynthesesystem.

Weiterhin betrifft die Erfindung eine Verwendung eines Streptavidin-Bindungspeptides zur Aufreinigung eines in einem Proteinbiosynthesesystem hergestellten definierten Proteins, sowie die Verwendung eines Streptavidin-Bindungspeptides zur Markierung eines definierten Proteins.

### Stand der Technik

Verfahren zur effizienten Expression von definierten Proteinen in verschiedensten pro- und eukaryontischen Organismen sind bekannt und bedürfen keiner weiteren Erläuterung. Unter definierten Proteinen werden in diesem Zusammenhang Peptide und/oder Proteine verstanden, die in dem zur Expression verwendeten Organismus oder zellfreien Expressionssystem natürlicherweise oder nach Transformation bzw. Einsatz definierter RNA exprimiert und in Aufreinigungsschritten angereichert werden.

Verfahren zur zellfreien Expression von definierten Proteinen sind beispielsweise aus den Literaturstellen EP 0312 617 B1, EP 0401 369 B1 und EP 0 593 757 B1 bekannt. Demgemäß werden die für eine Transkription und/oder Translation notwendigen Komponenten neben einem für ein definiertes Protein kodierenden Nukleinsäurestrang in einem Reaktionsgefäß inkubiert und nach der Expression die Polypeptide/Proteine aus der Reaktionslösung isoliert. Sowohl die für die Transkription, als auch die für die Translation notwendigen Komponenten lassen sich aus den Überstanden pro- oder eukaryontischen Zelllysaten nach einer Zentrifugation gewinnen.

Ein wesentliches Problem bei der Expression von definierten Proteinen in pro- und eukaryontischen Organismen und bei der zellfreien Expression liegt in der Aufreinigung und/oder der Detektion der exprimierten definierten Proteine. Dies ist insbesondere bei definierten Proteinen problematisch, für die es keine Antiseren oder monoklonale Antikörper gibt. Zur Vereinfachung der Aufreinigung und der Detektion solcher definierten Proteine werden diese als sogenannte Fusionsproteine exprimiert. Hierbei wird dem definierten Protein N- und/oder C-terminal eine Aminosäuresequenz angefügt oder zwischen zwei Proteindomänen (intern) eingefügt, der Fusionspartner. Dies geschieht auf der Nukleinsäureebene, so daß sowohl das definierte Protein, als auch der zur Detektion und/oder Reinigung angefügte Fusionspartner während eines Transkriptions-/Translationsvorganges zusammen als ein chimäres (Fusions-) Protein exprimiert werden, bestehend aus dem definierten Protein und dem Fusionspartner. Hierbei kann es sich bei dem angefügten Fusionspartner um einzelne Aminosäuren, aber auch um Peptide oder Proteine handeln. Für diese angefügten Fusionspartner stehen zur Aufreinigung immobilisierte Bindungspartner zur Verfügung, mit denen die Fusionsproteine isoliert werden können. Neben der Möglichkeit der Reinigung der Proteine können diese auch mit für den Fusionspartner spezifischen Bindungspartner nachgewiesen werden. Diese Bindungspartner können für den Fusionspartner spezifische Antikörper oder auch andere Proteine, Peptide oder chemische Verbindungen sein, die an den Fusionspartner spezifisch binden.

Beispiele für solche Fusionspartner sind der Myc-tag (Munro & Pelham (1986) Cell 46, 291-300; Ward et al. (1998) Nature 341, 544-546), das Flag Peptid (Hopp et al. (1988)Bio/Technology 6 1204-1210), das KT3 Epitop Peptid (Martinet et al. (1990) Cell 63, 843-849; Martin et al. (1992) Science 255, 192-194) und das alpha-tubulin Epitop Peptid (Skinner et al. (1991) J. Biol. Chem. 266, 14163-14166), die alle erfolgreich für die Detektion und teilweise auch für die Reinigung von Proteinen genutzt wurden. Es konnte für einen Teil der Fusionspartner, die normalerweise 3 bis 12 Aminosäuren lang sind, gezeigt werden, daß sie nicht die biologische Funktion der definierten Proteine beeinflussen. Die biologische Funktion des definierten Proteins wird dagegen mit zunehmender Länge des Fusionspartners beeinflußt, da die zusätzlich exprimierten Aminosäuren z. B. die Ausbildung der Sekunder-, Tertiär und/oder Quartärstruktur beeinflussen können. Längere Fusionspartner sind daher zur Detektion der Proteine, aber weniger zur Reinigung der Proteine geeignet. Auf der anderen Seite weisen längere Fusionspartner oft eine höhere Affinität mit ihren spezifischen Bindungspartnern auf.

Ein wesentlicher Nachteil der oben genannten Fusionspartner bei der Aufreinigung ist darin begründet, daß die Bindung an den Bindungspartner auf einer Antigen/Antikörperbindung beruht und die Herstellung und Reinigung der als Bindungspartner genutzten Antikörper aufwendig und teuer ist. Ein weitere Nachteil liegt darin begründet, daß die Antigen/Antikörperbindung eine sehr starke Bindung zwischen dem Bindungspartner, z. B. an eine Matrix immobilisierten Antikörper, und dem Fusionspartner bedingt. Diese hat zur Folge, daß bei der Elution der über den Fusionspartner gebundenen Fusionsproteine, teilweise extrem unphysiologische Bedingungen bezogen auf das definierte Protein geschaffen werden müssen. Unter unphysiologischen Bedingungen sind in diesem Zusammenhang Bedingungen zu verstehen mit z. B. sehr hohe oder äußerst geringe Salzkonzentrationen, Einsatz von chaotrophen Salzen und pH-Werte, die weit von dem natürlichen pH-Wert des definierten Proteins abweichen. Diese kann u. U. die Struktur und/oder Funktionalität des definierten Proteins beeinflussen oder irreversibel zerstören. Dementsprechend sollte die Reinigung der definierten Proteine unter möglichst schonenden, physiologischen Bedingungen geschehen, um die Funktionalität der Proteine zu erhalten. Zwar konnte bei drei der oben genannten Fusionspartnern (Hopp et al. (1988) (Martin et et al. (1990) (Skinner et al. (1991)) eine Elution auch unter schonenden Bedingungen mittels kompetetiver Peptide erzielt werden, doch bleibt das Problem der aufwendig und teuer herzustellenden und zu reinigenden (als Bindungspartner dienenden) Antikörper und deren Bindung an die Matrix.

Weitere Fusionspartner, bestehend aus 8 bis 9 Aminosäuren, sind aus den Literaturstellen US 5,506,121 und Schmidt & Skerra (Protein Engineering, vol. 6, no. 1, 109-122, 1993) bekannt. Die dort offenbarten Fusionspartner sind in der Lage an das Streptavidin oder an das "core" Streptavidin, ein proteolytisch gespaltenes Produkt des Streptavidin, zu binden (Bayer et al. (1989) Biochem. J. 259,369-376).

Alle bekannten Fusionspartner, die an das Streptavidin binden enthalten die Aminosäureabfolge HPQ, das sog. HPQ-Bindungsmotiv, welches mit der Biotin-Bindungstasche des Streptavidin in Wechselwirkung tritt. Zu den bereits bekannten Peptiden gehören dem sog. Strep-tag I: AWRHPQFGG mit einer Dissoziationskonstanten Kd von 10-37 µM, der sog. Strep-tag II: WSHPQFEK mit einer Dissoziationskonstanten Kd von 18-72 µM und der sog. SBP-tag: MDEKTTGWRGGHVVEGLAGELEQLRARLEHHPQGQREP mit einer Dissoziationskonstanten Kd von 2,5 nM. Im Gegensatz zu den beiden kurzen Strep-tags I und II besitzt der längere SBP-tag eine wesentlich stärkere Bindung zum Streptavidin. Allerdings wird, wie oben ausgeführt, die Funktion des definierten Proteins insbesondere mit zunehmender Länge des Fusionspartners beeinflußt. Auch stören besonders lange Fusionspartner bei der Kristallisation der definierten Proteine.

Strepavidin-Bindungspeptide mit HPQ-Motiv sind des weiteren bekannt aus den Literaturstellen Gene, Elsevier Biomedical Press. Amsterdam, Nl (1993), (128), 59-65; Febs Letters, Elsevier Science Publishers, Amsterdam, Nl (27-07-2001), 502(1-2), 35-40; Bioorganic & Medicinal Chemistry letters, Oxford, Gb (08-09-1998), 8(17), 2327-2332, Febs Letters (1995), 362, 306-308; Science, American Association For The Advancement Of Science, US (17-07-1990), 249, 404-406; Proceedings Of The National Academy Of Science Of USA, National Academy Of Science, Washington, US (27-03-2001), 98(7), 3750-3755; und Journal Of Molecular Biology, London, Gb (1996), 255 (5), 753-766.

### Technisches Problem der Erfindung

Der Erfindung liegt das technische Problem zugrunde, ein möglichst kurzes Streptavidin-Bindungspeptid mit einer starken Bindung zum Streptavidin zur Verfügung zu stellen.

### Grundzüge der Erfindung und bevorzugte Ausführungsformen.'

Zur Lösung des technischen Problems lehrt die Erfindung ein Streptavidin-Bindungspeptid gemäß Anspruch 1.

Mit dem erfindungsgemäßen Streptavidin-Bindungspeptid wird, verglichen mit dem Stand der Technik, eine wesentlich stärkere Bindung zwischen dem Streptavidin-Bindungspeptid und dem Streptavidin erreicht, bzw. kann bei gleicher Bindungsstärke das Streptavidin-Bindungspeptid wesentlich verkürzt werden.

Des Weiteren lehrt die Erfindung ein Nukleinsäure codierend für ein erfindungs gemäß Streptavidin-Bindungspeptid, sowie ein Plasmid enthaltend eine erfindungsgemäße Nukleinsäure. Es versteht sich, daß die Nukleinsäure codierend für ein erfindungsgemäßes Streptavidin-Bindungspeptid und/oder das Plasmid dem jeweiligen Expressionssystem/Proteinbiosynthesesystem angepaßt werden kann. Das Plasmid kann als ein Expressionsvektor, insbesondere für Bakterien, ausgelegt sein enthaltend einen Bereich mit mindestens einer Schnittstelle für ein Restriktionsenzym, in dem die Nukleinsäuresequenz codierend für ein definiertes Protein inseriert werden kann und somit das definierte Protein zusammen mit dem Streptavidin-Bindungspeptid exprimiert wird. Es versteht sich, daß der für das definierte Protein und für das Streptavidin-Bindungspeptide codierende Bereich sich unter der Kontrolle eines geeigneten Promoters und/oder Operators und Terminators befinden. Der Bereich mit mindestens einer Schnittstelle für mindestens ein Restriktionsenzym kann sowohl in 5', als auch in 3' Richtung vom Nukleinsäurebereich codierend für das Streptavidin-Bindungspeptide liegen. Der Nukleinsäurebereich codierend für das Streptavidin-Bindungspeptid muß nicht unmittelbar an den Nukleinsäurebereich codierend für das definierte Protein anschließen, vielmehr können zwischen den beiden Bereichen noch Nukleinsäuren liegen, die für 1 bis 20 Aminosäuren, insbesondere für 5 bis 10 Aminosäuren, codieren.

Die Erfindung betrifft auch ein Verfahren zur Herstellung eines erfindugsgemäßen Streptavidin-Bindungspeptides, wobei eine Nukleinsäure in einem zellbasierten oder zellfreien Proteinbiosynthesesystem exprimiert oder überexprimiert wird. Dieses so hergestellte Peptid läßt sich leicht über die Bindung an Streptavidin isolieren. Das erhaltene Translationsprodukt, i. e. das Streptavidin-Bindungspeptid, wird mit immobilisiertem Streptavidin kontaktiert und daran gebunden. Nach Abtrennung der Lösung mit nicht an Streptavidin gebundenen Substanzen wird das Translationsprodukt eluiert. Als Elutionsmittel können Puffer verwendet werden, die Biotin oder verwandte Substanzen und/oder Derivate, wie Iminobiotin, Desthiobiotin und/oder Diaminobiotin, enthalten. Das erhaltene Streptavidin-Bindungspeptid trägt im Falle der Fusion mit dem definierten Protein dieses Protein. Es kann der auch unabhängig von einem definierten Protein zur Antikörperherstellung genutzt werden. Die erhaltenen Antikörper können z. B. zur Detektion oder zur Aufreinigung der Streptavidin-Bindungspeptide, bzw. im Fall, daß das Streptavidin-Bindungspeptid als Fusionspartner eingesetzt wird, des an diesen Fusionspartner gebundenen definierten Proteins genutzt werden.

Es versteht sich, daß die Herstellung eines solchen Streptavidin-Bindungspeptides z.B. auch mittels chemischer Festphasensynthese, beispielseise mit einem Syntheseautomat der Firma Abimed (Langenfeld, BRD) möglich ist. Diese Methode basiert auf den Standardprotokollen der Fmoc-Chemie (Fmoc=9-fluorenylmethoxycarbonyl).

Die Erfindung betrifft auch die Verwendung eines erfindungs gemäßen Streptavidin-Bindungspeptides zur Aufreinigung eines in einem Proteinbiosynthesesystem hergestellten definierten Proteins, wobei eine für das definierte Protein und, hiermit verbunden, für das Streptavidin-Bindungspeptid codierende Nukleinsäure einer Transkription und/oder Translation unterworfen wird, wobei eine Lösung enthaltend das so erhaltene Translationsprodukt mit immobilisiertem Streptavidin kontaktiert und daran gebunden wird und wobei nach Abtrennung der Lösung mit nicht an Streptavidin gebundenen Substanzen das Translationsprodukt eluiert wird. Die Elution kann unter schonenden Bedingungen für das definierte Protein erfolgen. Als Elutionsmittel können Puffer verwendet werden, die Biotin oder verwandte Substanzen und/oder Derivate, wie Iminobiotin, Desthiobiotin und/oder Diaminobiotin, enthalten. Das hergestellte definierte Protein kann das als Fusionspartner dienende Streptavidin-Bindungspeptid sowohl N- und/oder C-terminal enthalten.

Bei Verwendung einer Streptavidin-Sepharose-Säule kann das zu untersuchende definierten Protein mittels des als Fusionspartner dienenden Streptavidin-Bindungspeptides an der Matrix immobilisiert und aus einem Gemisch von Molekülen, z.B. einem Zelllysat, isoliert werden.

Die Erfindung betrifft auch die Verwendung eines erfindungsgemäßen Streptavidin-Bindungspeptides zur Markierung eines definierten Proteins, wobei eine für das definierte Protein und, hiermit verbunden, für das Streptavidin-Bindungspeptid codierende Nukleinsäure einer Transkription und/oder Translation unterworfen wird, wobei das so erhaltene Translationsprodukt mit einem Streptavidin-Konjugat enthaltend ein Reportermolekül kontaktiert und daran gebunden wird. Das markierte definierte Protein kann das zur Markierung dienende Streptavidin-Bindungspeptides gemäß Seq.-ID 8 N- und/oder C-terminal enthalten. Reportermoleküle können z. B. radioaktive und/oder radioaktiv markierte Substanzen sein. Es versteht sich, daß das Streptavidin als solches auch radioaktiv markiert und/oder radioaktiv sein kann; in diesem Fall kann auf ein Reportermolekül verzichtet werden. Reportermoleküle können auch fluoreszierende Substanzen oder Enzyme, wie z. B. alkalische Phosphatase oder Peroxidase, sein. Durch solche mit einem Reportermolekül gekoppelte Streptavidinverbindungen lassen sich beispielsweise Proteine, die das Streptavidin-Bindungspeptid als Fusionspartner besitzen, auf einem Western-Blot oder im ELISA (Enzyme linked immunosorbent assay) nachweisen und/oder quantifizieren. Handelt es sich bei den definierten Proteinen um Bindungsproteine, können auf diese Weise auch an diese bindende andere Proteine nachgewiesen werden. Unter Bindungsproteine sind in diesem Zusammenhang Proteine zu verstehen, die selbst andere Proteine binden und/oder selber an andere Proteine binden, wie z. B. bei einer Antigen/Antikörperbindung oder bei einer Bindung eines Proteins an einen Rezeptor.

Bevorzugt ist ein Streptavidin-Bindungspeptid enthaltend weniger als 30 Aminosäuren, vorzugsweise weniger als 20 Aminosäuren, höchstvorzugsweise weniger als 10 Aminosäuren.

Im Folgenden wird die Erfindung anhand von lediglich Ausführungsformen darstellenden Figuren sowie Beispielen näher erläutert.

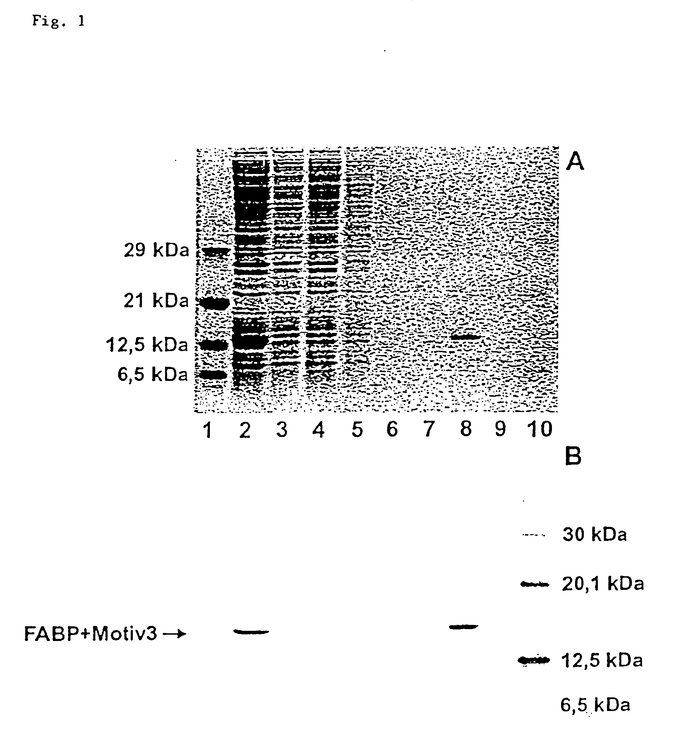

- Fig. 1:: Reinigung von FABP mit Streptavidin-Bindungspeptides gemäß Motiv 3 als Fusionspartner nach zellfreier Proteinbiosynthese über eine StreptavidinAffinitätssäule. Es wurden von jeder Fraktion eine vergleichbare Menge mittels SDS-Polyacrylamidgelelektrophorese analysiert. (A) Coomassie gefärbt und (B) Autoradiogramm. Die Proben in den numerierten Spuren sind (1) Molekulargewichtsmarker; (2) Reaktionsmischung; (3) Durchlauf der Probenauftragung; (4-6) Waschfraktionen; (7-9) Elutionsfraktionen; (10) radioaktiver Molekulargewichtsmarker.

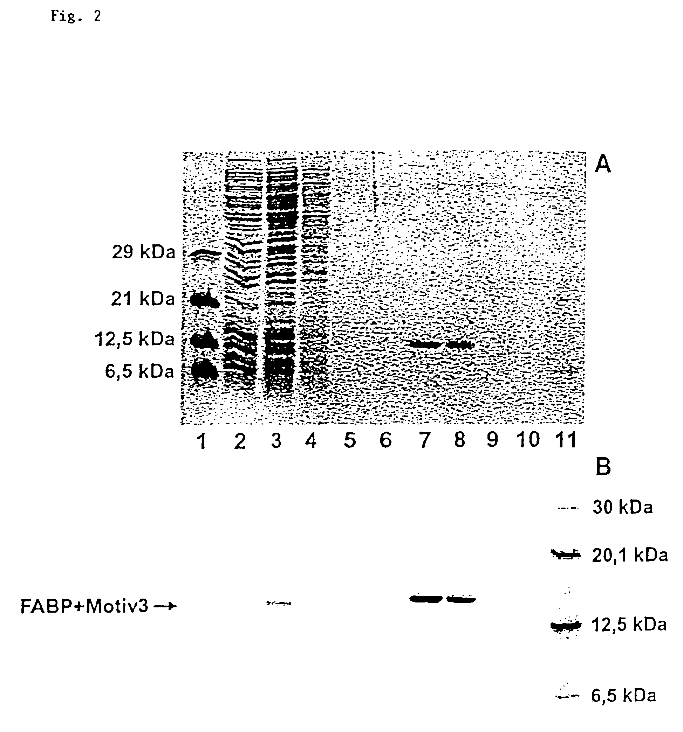

- Fig. 2:: Reinigung von FABP mit Streptavidin-Bindungspeptides gemäß Motiv 3 als Fusionspartner nach zellfreier Proteinbiosynthese über eine StrepTactinAffinitätssäule. Es wurden von jeder Fraktion eine vergleichbare Menge mittels SDS-Polyacrylamidgelelektrophorese analysiert. (A) Coomassie gefärbt und (B) Autoradiogramm. Die Proben in den numerierten Spuren sind (1) Molekulargewichtsmarker; (2) Durchlauf der Probenauftragung; (3-5) Waschfraktionen; (6-10) Elutionsfraktionen; (11) radioaktiver Molekulargewichtsmarker.

### Beispiel 1 : erfindungsgemäße Bindungspeptide und Vergleichspeptide mit Dissoziationskonstanten

In fachüblicher Weise wurde das FAB Protein (fatty acid binding protein) aus Rinderherzen *in vitro* in einem zellfreien Proteinbiosynthese mittels eines gekoppelten Transkriptions-Translationssystemes exprimiert. Das exprimierte FAB Protein besaß am N-Terminus jeweils ein aus fünfzehn Aminosäuren bestehendes zusätzliches Streptavidin Bindungspeptid mit unterschiedlichen Aminosäuresequenzen als Fusionspartner.

Zwei Bindungspeptide enthalten in ihrer Aminosäuresequenz das im Stand der Technik beschriebene HPQ-Motiv(Motiv 1 und 2) und zwei enthalten das erfindungsgemäße Streptavidin-Bindungspeptid (Motiv 3). Die Sequenzen der Peptide sind in der Tabelle 1 dargestellt. Zusätzlich wurden die exprimierten Proteine mit einem am C-terminus befindlichen "His-tag", bestehend aus 6 Histidinen, versehen. Die Proteine wurden mittels Affinitätschromatographie über Ni²⁺-IDA-Agarose in fachüblicher Weise aufgereinigt. Man erkennt, dass bei gleicher Länge Bindungspeptide mit einer erfindungsgemäßen Sequenz eine wesentlich niedrigere Dissoziationskonstante als ein Bindungspeptid mit HPQ-Motiv aufweist. Der Kd-Wert eines erfindunggemäßen Binduhgspeptids liegt in der Größenordnung des SBP-Tags trotz der ca. 2,5-fachen Länge des SBP-Tags.

**Tabelle 1**

| | Sequenz | Dissoziations-konstante [kd] |
|---|---|---|
| Motiv 1 | D L Y D I D R N W V G H P Q G | 8 µM |
| Motiv 2 | D N Y D A D L A W D T H P Q D | 70 µM |
| Motiv 3 | D V E A W L D E R V P L V E T | 84 nM |

### Beispiel 2: Messungsweise der Dissoziationskonstanten aus Tabelle 1.

Die Messungen der Dissoziationskonstante wurden mit einem BiacoreX-System und dem Sensor Chip NTA der Firma Biacore durchgeführt. Vermessen wurden die aus der im Beispiel 1 beschriebenen Expression hervorgegangenen Proteine, d.h. FAB Proteine, die am N-Terminus jeweils ein aus fünfzehn Aminosäuren bestehendes Peptid als Fusionspartner und am C-Terminus 6 Histidine besaßen, die zur Immobilisierung auf dem Sensor Chip benötigt wurden. Die Bindungsaffinität der aus der im Beispiel 1 beschriebenen Expression hervorgegangenen Proteine zu Streptavidin wurde nach Herstellerangaben im Biacore-Gerät vermessen. Dabei befindet sich das zu vermessende Protein auf dem Sensor-Chip und eine Streptavidinlösung mit definierter Konzentration wird eingespritzt. Die Wechselwirkung (Bindung) zwischen den beiden Molekülen wird vom Gerät gemessen und als sog. Resonanz Units (RU) angegeben. Zur Messung wurden die vom Hersteller angegebenen Puffer verwendet.

Die Ergebnisse der Messungen sind in der Tabelle 2 dargestellt. Die erhaltenen Meßwerte wurden mit der zugehörigen Software ausgewertet und führten zu den in der Tabelle 1 angegebenen Dissoziationskonstanten.

**Tabelle 2:**

| Motiv | 1 | 2 | 3 |
|---|---|---|---|
| Streptavidinkonz. | RU | RU | RU |
| 15 nM | | | 98 |
| 30 nM | | | 242 |
| 60 nM | | | 461 |
| 125 nM | | | 613 |
| 250 nM | | | 704 |
| 500 nM | 62 | | 786 |
| 1 µM | 123 | | |
| 2 µM | 233 | | 946 |
| 3 µM | - | 64 | - |
| 4 µM | 407 | - | 983 |
| 6 µM | - | 98 | |
| 8 µM | 621 | - | |
| 15 µM | - | 201 | |
| 16 µM | 779 | - | |
| 30 µM | - | 337 | |
| 32 µM | 955 | - | |
| 60 µM | | 536 | |

### Beispiel 3: Aufreinigung eines Fusionsproteins

In fachüblicher weise wurde das FAB Protein, das am N-terminus ein aus fünfzehn Aminosäuren bestehendes zusätzliches Peptide mit der Aminosäuresequenz D V E A W L D E R V P L V E T (Motiv 3) als Fusionspartner besaß, *in vitro* in einem zellfreien Proteinbiosynthese mittels eines gekoppelten Transkriptions-Translationssystems exprimiert. Zur Aufreinigung des überexprimierten definierten Proteins wurde das Streptavidin an einer Festphase gekoppelt. Als Festphase diente eine Sepharose. Das exprimierte FAB Protein wurde anschließend in fachüblicher weise affinitätschromatographisch über eine Säule enthaltend Streptavidin-Sepharose oder StrepTactin-Sepharose aufgereinigt. Für die Aufreinigung wurden folgende Puffer verwendet: Waschpuffer (100 mM Tris-HCl (pH 8.0), 150 mM NaCl, 1 mM EDTA).

Für die Elution von Streptavidin enthielt der Waschpuffer 2 mM Biotin und für die Elution von StrepTactin enthielt der Waschpuffer 2,5 mM Desthiobiotin.

Die prozentuale Verteilung des eingesetzten definierten Proteins auf die verschiedenen Fraktionen der Affinitätschromatographie ist in der Tabelle 3 zu entnehmen. In der Tabelle 3 steht D für den Auftragung der Probe/Durchlauf, W für die Waschfraktion und E für die Elutionsfraktion.

**Tabelle 3**

| Fraktion | D | W1 | W2 | W3 | E1 | E2 | E3 | E4 | E5 | Summe |
|---|---|---|---|---|---|---|---|---|---|---|
| Strept-avidin | 3,5% | 6,8% | 1,0% | 0,3% | 0,4% | 76,9% | 0,7% | - | - | 89,6% |
| StrepT-actin | 3,0% | 7,4% | 1,8% | 0,9% | 0,9% | 45,9% | 23,2% | 1,0% | 0,3% | 84,4% |

Bei der Verwendung von Streptavidin-Sepharose konnten 90% des aufgetragenen Proteins von der Säule wiedergewonnen werden, wobei 78% auf das Eluat entfielen. Die Qualität der Aufreinigung ist in Fig. 1 dargestellt.

Bei der Verwendung von StrepTactin-Sepharose konnten 84% des aufgetragenen Proteins von der Säule wiedergewonnen werden, wobei, 71% auf das Eluat entfielen. Die Qualität der Aufreinigung ist in Fig. 2 dargestellt.

### Beispiel 4: Optimierung einzelner Bindungspeptide

Das Bindungspeptid (FAx 3) mit der Sequenz DVEAWLDERVPLVET wurde einer Substitutionsanalyse unterzogen, wodurch ein etwaiges Minimalmotiv (=verkürztes Peptid) identifiziert und die Wirkung einzelner Aminosäureaustausche auf die Bindungsspezifität untersucht werden sollten. Bei einer Substitutionsanalyse werden Peptide mittels Spot-Verfahren (Frank, R. 1992) auf einer als Festphase dienenden Cellulosemembran synthetisiert. Dabei wurde jede Position des 15 Aminosäuren umfassenden Peptids systematisch durch die übrigen 19 L-Aminosäuren ersetzt.

Die Substitutionsanalyse wurde mit einem Peroxidase-markierten Streptavidin inkubiert und mit einem Lumineszenzsubstart entwickelt. Dadurch konnte festgestellt werden wie weit sich das Peptid verkürzen läßt und welche Aminosäuren "konserviert" und somit nicht oder nur sehr schlecht austauschbar sind. Es ergab sich folgendes Bild: Ein Peptid, das hinreichende Streptavidinbindung aufweist ist das 6-mer Peptid DVEAWL. Eine deutliche bessere Streptavidinbindung weist das 9-mer Peptid DVEAWLDER auf. Die Substitutionsanalyse wies auch auf Positionen hin, wo der Austausch der ursprünglichen gegen eine andere Aminosäure eine Verbesserung der Streptavidinbindung mit sich bringen könnte.

Die Informationen aus der Substitutionsanalyse wurden überprüft, indem die Dissoziations-konstanten verschiedener Peptide mittels "Oberflächen Plasmonen Resonanz" Spektroskopie unter Verwendung eines BIAcore-Gerätes bestimmt wurden. Die Messungen erfolgten mit dem NTA Sensor-Chip der Firma BIAcore und mit Hilfe des fettsäurebindenden Proteins aus dem Rinderherzen (FABP). Das FABP diente zur Immobilisierung und Präsentation der Peptide. Weiterhin sollten so die bei einer Affinitätschromatographie auftretenden Bedingungen simuliert werden, da das Peptid zukünftig als Affinitätspeptid zur Aufreinigung von Proteinen verwendet werden soll. Im Gegensatz zu den ersten Messungen, wo die zu vermessenden Peptide die vier N-terminalen Aminosäuren des FABP ersetzten, war die Anordnung der Peptide diesmal ebenfalls N-terminal, aber sie waren nicht Teil des FABP. Auch der für die Immobilisierung notwendige His-tag wurde verändert. Er befand sich wie bei den ersten Messungen am C-Terminus des FABP, wurde aber von sechs auf acht Histidine erweitert und zudem durch einen aus zwei Glycinen bestehenden Linker vom FABP distanziert. Dadurch konnte die Immobilisierung der Liganden deutlich verbesssert und somit die Drift der Basislinie, die aus dem Herunterwaschen des Liganden resultiert, minimiert werden. Für jede Messung wurde eine identische Menge des jeweiligen FABPs immobilisiert, die einem Anstieg der Basislinie um 1100 Resonanzeinheiten (RU) entsprach. Als Referenz diente das FABP mit dem His-tag, aber ohne N-terminales Bindungspeptid.

Die aus der optimierten Meßmethode hervorgehenden Dissoziateonskonstanten waren folgende:

| | **Kd** |
|---|---|
| Peptid (FAx 3): | 3,6±0,6 nM |
| | |
| verkürztes Peptid (9-mer): DVEAWLDER | 240± 40 nM |

Einfluß anderer Aminosäuren auf das verkürzte 9-mer Peptid:

| | |
|---|---|
| 3 Asp: DV**D**AWLDER | 940± 140 nM |
| 3 Gly: DV**G**AWLDER | 570± 90 nM |
| 6 Phe: DVEAW**F**DER | nicht meßbar |
| 6 Arg: DVEAW**R**DER | nicht meßbar |
| 7 Gly: DVEAWL**G**ER | 17± 4 nM |
| 8 Ala: DVEAWLD**A**R | 160± 30 nM |

Positiven Einfluß zeigen insbesondere Glycin an Position 7 und Alanin an Position 8. Kombination dieser beiden Aminosäuren:

| | |
|---|---|
| verkürztes Peptid: DVEAWL**GA**R | 17± 4 nM |
| original Peptid mit Gly7: DVEAWL**G**ERVPLVET | 4,2± 0,7 nM |
| original Peptid: DVEAWL**GA**RVPLVET | 4,1± 0,6 nM |

Das original 15-mer Peptid läßt sich nicht weiter verbessern. Das verkürzten 9-mer Peptid hingegen läßt sich durch ein Glycin anstelle der Asparaginsäure an Position sieben deutlich verbessern. Auch das Alanin anstelle der Glutaminsäure an Position acht weist eine etwas bessere Bindungsaffinität gegenüber Streptavidin auf. Durch die Kombination dieser beiden Aminosäuren läßt sich zwar die Bindungsaffinität nicht weiter verbessern, aber sie verschlechtert sich auch nicht.

Die Erfindung umfasst somit auch ein 9-mer Peptid mit seinen nicht bzw. nur wenig konservierten Positionen:
DVXAWLXXR (X= beliebeige Aminosäure)
Zudem natürlich das verkürzte Peptid mit Glycin an Position sieben:
DVEAWLGER
und das verkürzte Peptid mit Glycin an Position sieben und Alanin an Position acht:
DVEAWLGAR

### Beispiel 5: Varianten erfindungsgemäßer Sequenzen.

Folgend werden geeignete Sequenzen angegeben.

| | | |
|---|---|---|
| Seq.-ID 1: | DVEAW | (nicht vorliegende Erfindung) |
| Seq.-ID 2: | DVEA | (nicht vorliegende Erfindung) |
| Seq.-ID 3: | VEAW | (nicht vorliegende Erfindung) |
| Seq.-ID 4: | DVE | (nicht vorliegende Erfindung) |
| Seq.-ID 5: | VEA | (nicht vorliegende Erfindung) |
| Seq.-ID 6: | EAW | (nicht vorliegende Erfindung) |
| Seq.-ID 7: | DVXAW | (nicht vorliegende Erfindung) |
| Seq.-ID 8: | DVXAWL | (erfindungsgemäß) |
| Seq.-ID 9: | DVXAWLX | (erfindungsgemäß) |
| Seq.-ID 10: | DVXAWLXX | (erfindungsgemäß) |
| Seq.-ID 11: | DVXAWLXXR | (erfindungsgemäß) |
| Seq.-ID 12: | DVXAWLXXRVPLVET | (erfindungsgemäß) |
| Seq.-ID 13: | VPLVET | (erfindungsgemäße Verlängerung) |

X an Position 3 kann beliebig cauber 2,5, order n), jedoch insbesondere E, D oder G sein. X kann an Position 7 beliebig, jedoch insbesondere G oder D sein. X kann an Position 8 beliebig, jedoch insbesondere A oder E sein. Sequenz 13 stellt eine optionale carboxyterminale Verlängerung der Sequenz 11 dar, wobei aus Sequenz 13, aminoterminal beginnend 1 bis 6 Aminosäuren an Sequenz 11 angeschlossen sein können.

## Patentansprüche

1. Streptavidin-Bindungspeptid enthaltend eine oder bestehend aus einer Aminosäuresequenz gemäß Seq.-ID 8, wobei Xaa nicht Leu, Met, oder Ser ist, wobei das Streptavidin-Bindungspeptid weniger als 30 Aminosäuren enthält.

2. Nukleinsäure codierend für ein Streptavidin-Bindungspeptid nach Anspruch 1.

3. Plasmid enthaltend eine Nukleinsäure nach Anspruch 2.

4. Verfahren zur Herstellung eines Streptavidin-Bindungspeptides nach Anspruch 1, wobei eine Nukleinsäure nach Anspruch 2 in einem zellbasierten oder zellfreien Proteinbiosynthesesystem exprimiert oder überexprimiert wird.

5. Verwendung eines Streptavidin-Bindungspeptides nach Anspruch 1 zur Aufreinigung eines in einem Proteinbiosynthesesystem hergestellten definierten Proteins, wobei eine für das definierte Protein und, hiermit verbunden, für das Streptavidin-Bindungspeptid codierende Nukleinsäure, optional kontrolliert durch eine regulatorische Sequenz, einer Transkription und/oder Translation unterworfen wird, wobei eine Lösung enthaltend das so erhaltene Translationsprodukt mit immobilisiertem Streptavidin kontaktiert wird und wobei nach Abtrennung der Lösung mit nicht an Streptavidin gebundenen Substanzen von dem Streptavidin das Translationsprodukt vom Streptavidin freigesetzt und eluiert wird.

6. Verwendung eines Streptavidin-Bindungspeptides nach Anspruch 1 zur Markierung eines definierten Proteins, wobei eine für das definierte Protein und, hiermit verbunden, für das Streptavidin-Bindungspeptid codierende Nukleinsäure einer Transkription und/oder Translation unterworfen wird, wobei das so erhaltene Translationsprodukt mit einem Streptavidin-Konjugat enthaltend ein Reportermolekül kontaktiert und daran gebunden wird.

## Claims

1. A streptavidin-binding peptide containing or composed of an amino acid sequence according to Seq.-ID 8, wherein Xaa is not Leu, Met, or Ser, wherein the streptavidin-binding peptide contains less than 30 amino acids.

2. A nucleic acid coding for a streptavidin-binding peptide according to claim 1.

3. A plasmid containing a nucleic acid according to claim 2.

4. A method for producing a streptavidin-binding peptide according to claim 1, wherein a nucleic acid according to claim 2 is expressed or over-expressed in a cell-based or cell-free protein biosynthesis system.

5. The use of a streptavidin-binding peptide according to claim 1 for purifying a defined protein produced in a protein biosynthesis system, wherein a nucleic acid coding for the defined protein and thus for the streptavidin-binding peptide, optionally controlled by a regulatory sequence, is submitted to a transcription and/or translation, wherein a solution containing the thus obtained translation product is contacted with immobilized streptavidin, and wherein after separation of the solution from the streptavidin with substances not bound to streptavidin, the translation product is released from the streptavidin, and is eluated.

6. The use of a streptavidin-binding peptide according to claim 1 for marking a defined protein, wherein a nucleic acid coding for the defined protein and thus for the streptavidin-binding peptide is submitted to a transcription and/or translation, wherein the thus obtained translation product is contacted with a streptavidin conjugate containing a reporter molecule, and is bound thereto.

## Revendications

1. Peptide liant la streptavidine contenant ou constitué d'une séquence aminoacide selon la SEQ.-ID 8, dans lequel Xaa n'est pas Leu, Mét ou Sér, dans lequel le peptide liant la streptavidine contient moins de 30 acides aminés.

2. Acide nucléique codant pour un peptide liant la streptavidine selon la revendication 1.

3. Plasmide contenant un acide nucléique selon la revendication 2.

4. Procédé de production d'un peptide liant la streptavidine selon la revendication 1, dans lequel un acide nucléique selon la revendication 2 est exprimé ou surexprimé dans un système de biosynthèse des protéines basé sur cellules ou sans cellules.

5. Utilisation d'un peptide liant la streptavidine selon la revendication 1 pour la purification d'une protéine définie produite dans un système de biosynthèse des protéines, dans laquelle un acide nucléique codant pour la protéine définie et donc pour le peptide liant la streptavidine, optionnellement commandé par une séquence régulatrice, est soumis à une transcription et/ou traduction, dans laquelle une solution contenant le produit de traduction tellement obtenu est contacté avec de la streptavidine immobilisée, et dans laquelle après la séparation de la solution de la streptavidine avec des substances non liées à la streptavidine, le produit de traduction est libéré de la streptavidine et est élué.

6. Utilisation d'un peptide liant la streptavidine selon la revendication 1 pour le marquage d'une protéine définie, dans laquelle un acide nucléique codant pour la protéine définie et donc pour le peptide liant la streptavidine est soumis à une transcription et/ou traduction, dans laquelle le produit de traduction tellement obtenu est contacté avec un conjugué de la streptavidine contenant une molécule rapporteur et est lié à celui-ci.
